# EUROPEAN PATENT APPLICATION

(11) **EP 4 650 451 A1**
(43) Date of publication of application: **19.11.2025**
(21) Application number: 24755821.6
(22) Date of filing: 03.01.2024
(51) Int. Cl.: C12N 15/62, C12N 15/85, C12N 5/10, A61K 48/00

(54) **MRNA MOLECULE FOR TARGETED PROTEIN DEGRADATION, AND USE**

(30) Priority: 17.02.2023 CN 202310127782
(71) Applicant: Cosychem Biotechnology Technology (Tianjin) Co., Ltd., Binhai New Area, Tianjin 300000 (CN)
(72) Inventor: TAN, Ying, Shenzhen, Guangdong 518055 (CN); JIANG, Yuyang, Shenzhen, Guangdong 518055 (CN); XUE, Xiaoqi, Shenzhen, Guangdong 518055 (CN); TAN, Chunyan, Shenzhen, Guangdong 518055 (CN); XU, Naihan, Shenzhen, Guangdong 518055 (CN)
(74) Representative: Lorenz Seidler Gossel Part. mbB
(86) International application number: PCT/CN2024/070318
(87) International publication number: WO 2024/169445

(57) **Abstract**

An mRNA molecule for targeted protein degradation and a use. The mRNA molecule for targeted protein degradation successively comprises a 5 'cap structure, a 5' non-coding region, a Kozak element, a coding region, a 3' non-coding region, and a polyadenylic acid structure; the coding region comprises a functional polypeptide coding gene, and the functional polypeptide comprises a VHL E3 ubiquitin ligase targeting peptide, a linker peptide, and a protein of interest targeting polypeptide that are successively connected. The mRNA molecule for targeted protein degradation has higher safety, can improve the anti-tumor efficacy of the peptide-based PROTACs, solve the critical cell penetration problem, and can be used for efficient targeted degradation of a protein of interest, provides a new targeted protein degradation molecular pattern, and has a broad application prospect.

## Description

### Technical Field

The present invention relates to an mRNA molecule for targeted protein degradation and its use in the biomedical field.

### Background Art

In countries around the world, cancer is a leading cause of death and a significant obstacle to increase human life expectancy. At present, there are various treatment strategies for cancer, mainly including chemotherapy, surgery, and radiotherapy. Chemotherapy has been remained as the main treatment for cancer since the 1940s, and anti-cancer drugs are currently the focus of drug development. However, chemotherapy often comes with some toxic and side effects, which are usually caused by the adverse effects of anti-cancer drugs on hematopoietic cells in the bone marrow, hair follicles, cells in the digestive tract and reproductive system. Therefore, the development of anti-cancer drugs is dedicated to improving non-specific and targeted properties.

During a process of tumor formation, due to significant changes in gene expression levels compared to normal cells, abnormal protein expression occurs, such as high levels of growth factor receptor expression. Overexpression of proteins is causally related to tumor formation. Overexpressed proteins in tumor cells can serve as tumor markers for accurate diagnosis of early cancer, as well as targets for anti-tumor drugs to overcome. Breast cancer is a common cancer. Among them, a type that estrogen receptor α (ERα) signaling pathway promotes the growth of breast cancer is breast cancer with the highest incidence, accounting for about 70% of the total number of patients. Therefore, ER α has become an important target for treatment of breast cancer. For decades, estrogen inhibitors and ER α antagonists have become the main treatment of ER⁺breast cancer.

Targeted protein degradation is one of the commonly used strategies in tumor therapy. Ubiquitination degradation of intracellular protein is a necessary pathway for maintaining cellular functional balance, and its application in tumor therapy has led to currently representative proteolysis-targeting chimeras (PROTACs) technology. PROTACs can degrade proteins of interest by recruiting cells' natural degradation tool, ubiquitin-proteasome system (UPS). PROTACs have great potential as they can degrade undruggable proteins and non-enzymatic proteins that traditional small molecule inhibitors cannot address, improving the shortcomings of small molecule inhibitors used in chemotherapy over the past few decades, namely non-specificity and drug resistance. The relevant reports and clinically validated data provide impetus for the development of this technology. With more and more attention being paid to PROTACs, as a promising technology, it is not only expanding in target range and molecular design, but also constantly innovating in environmental responsiveness and tumor targeting. This technology has been developed for 20 years, mainly in the form of chemical small molecules and peptide mimetic molecules. In recent years, biomolecules represented by nucleic acid molecules have emerged as ligands, emphasizing a need to pay attention to biocompatibility and safety of PROTACs.

With a success of mRNA vaccines developed by Moderna and Pfizer/BioNTech for COVID-19, mRNA technology has received unprecedented attention. mRNA vaccines have the potential for low-cost manufacturing and greater safety. It is because of these advantages that Moderna designed and produced the SARS-CoV-2 mRNA vaccine (mRNA-1273) for human use in just 42 days after obtaining a nucleotide sequence of the target antigen. More noteworthy is that the SARS-CoV-2 mRNA vaccine produced by Moderna and Pfizer/BioNTech has shown very high efficacy in phase III clinical trials and the general population (approximately 90% at ≤ 6 months of follow-up).

The expansion of mRNA applications is closely related to continuous improvement of in vitro transcribed mRNA (IVT-mRNA) technology, which enhances the stability and translation efficiency of mRNA in cells. Compared with subunit vaccines, inactivated and attenuated virus vaccines, as well as DNA vaccines, the use of IVT-mRNA has the following advantages: safety, mRNA is immediately translated in the cytoplasm, directly translated into proteins of interest through cellular mechanisms, without entering into the nucleus, so there is no potential risk of infection or insertion mutagenesis; stability, various modifications make mRNA more stable with higher translation efficiency; low cost, mRNA has the advantages of fast, cheap, and mass production. In addition to its use as a vaccine, mRNA is also applied in protein replacement therapy, but its potential goes far beyond that. Therefore, with the rapid improvement of in vitro transcribed mRNA technology, the development of new mRNA based therapies will be crucial in the future.

### Summary of the Invention

The development of anti-cancer drugs is a long-term task, especially in improving the specificity and targeting of anti-cancer drugs. The present invention provides an mRNA molecule (referred to as m-PROTAC) for targeted protein degradation, which sequentially comprises a 5' cap structure, a 5' non-coding region, a Kozak element, a coding region, a 3' non-coding region, and a polyadenylic acid structure. The coding region comprises a functional polypeptide-coding gene, and the functional polypeptide comprises a VHL E3 ubiquitin ligase targeting peptide, a linking peptide, and a protein of interest targeting polypeptide that are sequentially connected. The mRNA molecule of the present invention has good targeting and good therapeutic effects on tumors.

### Technical Problem

The technical problem to be solved by the present invention is to provide an mRNA system based on degradation of proteins of interest by PROTACs.

### Technical solutions

To solve the above technical problem, an mRNA molecule of the present invention, named m-PROTAC, sequentially comprises a 5' cap structure (5'-cap), a 5' non-coding region, a Kozak element, a coding region, a 3' non-coding region, and a polyadenylic acid structure. The coding region comprises a functional polypeptide-coding gene, and the functional peptide comprises a VHL E3 ubiquitin ligase targeting peptide, a linker peptide, and a protein of interest targeting polypeptide that are connected in sequence.

The core of the present invention is to provide a new system, namely an mRNA system based on PROTACs, specifically referring to module settings of the above mRNA molecule. Among them, 5'-cap, Kozak element, and poly-A are all conventional components of eukaryotic mRNA, Kozak element has a sequence shown as positions 51 to 56 of SEQ ID No. 7, and the 5' non-coding region and 3' non-coding region can be various conventional sequences in the art. In the present invention, 5' non-coding region of β - globin (sequence shown as positions 1 to 50 of SEQ ID No. 7) and 3' non-coding region of α-globin (sequence shown as positions 897 to 1007 of SEQ ID No. 7) are used. The coding region is an element used for degrading a protein of interest, based on the principle of PROTACs, it includes a VHL E3 ubiquitin ligase targeting peptide, a linking peptide, and a protein of interest targeting polypeptide. The VHL E3 ubiquitin ligase targeting peptide sequence is a well-known sequence, as shown in SEQ ID No.1. The linking peptide can be selected as a conventional sequence, such as the sequence shown in SEQ ID No.2 or SEQ ID No.3. The protein of interest targeting polypeptide is a polypeptide that specifically targets the protein of interest, which is selected or designed based on the protein of interest, and according to the principle of PROTACs, the specific sequence of which does not affect the function of the system.

According to one embodiment of the present invention, the protein of interest is estrogen receptor alpha, and a sequence of the corresponding protein of interest targeting polypeptide is shown in SEQ ID No. 4. Correspondingly, the sequence of the functional polypeptide is shown in SEQ ID No. 6.

According to another embodiment of the present invention, the protein of interest is an antiapoptotic protein BCL-x_{L}, and a sequence of the corresponding protein of interest targeting peptide is shown in SEQ ID No. 5. Correspondingly, the sequence of the functional polypeptide is shown in SEQ ID No. 11.

In the present invention, to verify the feasibility of the system, a reporter protein is designed, located in a coding region, and a sequence for spacing is set between the coding region and the functional polypeptide to avoid mutual influence of protein expression. As long as an appropriate spacer is set, the coding region can contain coding genes for other proteins, such as another functional protein coding gene or reporter protein coding gene. The coding region can be composed of the functional polypeptide coding gene, another functional protein coding gene or a reporter protein coding gene, as well as a spacer region set between them and the functional polypeptide.

According to a specific embodiment of the present invention, the reporter protein is EGFP (sequence is shown in positions 57-773 of SEQ ID No. 7), and the spacer region is a P2A peptide coding gene (sequence is shown in positions 774-839 of SEQ ID No. 7).

Based on the above design principles, when the protein of interest is an estrogen receptor alpha, a sequence of the mRNA molecule can be as shown in SEQ ID No.7. When the protein of interest is BCL-x_{L}, a sequence of the mRNA molecule can be as shown in SEQ ID No. 12.

The present invention also provides biomaterials related to the mRNA molecule. The second aspect of the present invention provides a DNA molecule encoding the mRNA molecule.

When the protein of interest is an estrogen receptor alpha, a sequence of the DNA molecule can be as shown in SEQ ID No. 8. When the protein of interest is BCL-x_{L}, a sequence of the DNA molecule can be as shown in SEQ ID No. 13.

The third aspect of the present invention provides a recombinant vector comprising the aforementioned DNA molecule. The vector can be a plasmid, cosmid, bacteriophage, or viral vector.

When the protein of interest is an estrogen receptor alpha, a sequence of the recombinant vector can be as shown in SEQ ID No. 9. When the protein of interest is BCL-x_{L}, a sequence of the recombinant vector can be as shown in SEQ ID No. 14. The fourth aspect of the present invention provides a recombinant microorganism transfected with (i.e. containing) the aforementioned mRNA molecule, DNA molecule, or recombinant vector. The microorganisms can be yeast, bacteria, algae, or fungi.

The fifth aspect of the present invention provides a cell transfected with (i.e. containing) the aforementioned mRNA molecule, DNA molecule, recombinant vector, or recombinant microorganism.

The material may or may not include reproductive materials.

According to the present invention, the cell can be a MCF-7 cell or a MDA-MB-231 cell.

The present invention also provides uses of any of the aforementioned mRNA molecule, DNA molecule, recombinant vector, or recombinant microorganism:
M1) preparation of a product for treating and/or prevention of diseases/lesions mediated by a protein of interest;
M2) treatment and/or prevention of diseases/lesions mediated by a protein of interest;
M3) preparation of a product that inhibits cell proliferation;
M4) inhibition of cell proliferation;
M5) preparation of a product that promotes cell apoptosis;
M6) promotion of cell apoptosis.

In the above uses, the product can be a vaccine or a medicament.

According to the present invention, the diseases mediated by the protein of interest can be cancer; the lesions mediated by the protein of interest can be plant or animal lesions.

According to the present invention, the cell can be a cancer cell.

The cancer include but are not limited to, a leukemia cancer, a lymphoma, a lung cancer, a breast cancer, an ovarian cancer, a cervical cancer, a human glioma, a melanoma cancer, a glioblastoma, a nasopharyngeal cancer, a liver cancer, a brain cancer, a pancreatic cancer, an uterine cancer, a testicular cancer, a skin cancer, a stomach cancer, a colon cancer, a bladder cancer or a rectal cancer.

In one embodiment of the present invention, the cancer is a breast cancer, the cell is a breast cancer cell, and the breast cancer cell is a MCF-7 cell.

In another embodiment of the present invention, the breast cancer cell is a MDA-MB-231 cell.

In still another embodiment of the invention, the breast cancer cell is a 4T1 cell line. The present invention also provides a method for degrading a protein of interest for non-therapeutic or therapeutic purposes, comprising: administrating the mRNA molecule to a biological cell, tissue or organ expressing the protein of interest to achieve degradation of the protein of interest in the biological cell, tissue or organ. The biological cell can be a microbial cells, a plant cell, or an ex vivo animal cell. The microbial cell can be yeast, bacteria, algae, or fungi. The animal cell can be a cancer cell, including but not limited to the following cancer cells: leukemia cancer, lymphoma, lung cancer, breast cancer, ovarian cancer, cervical cancer, human glioma, melanoma, glioblastoma, nasopharyngeal cancer, liver cancer, brain cancer, pancreatic cancer, uterine cancer, testicular cancer, skin cancer, stomach cancer, colon cancer, bladder cancer or rectal cancer.

The tissue is a plant tissue or an ex vivo animal tissue.

The organ is a plant organ or an ex vivo animal organ.

The present invention also provides a method for treating and/or preventing a diseases/lesion mediated by a protein of interest, comprising: administering the mRNA molecule to an animal body suffering from the disease/lesion mediated by the protein of interest, to achieve treatment and/or prevention and/or alleviation of the disease/lesion mediated by the protein of interest in the animal body.

The animal body can be a human or non-human animal body.

In the above methods, the disease mediated by the protein of interest can be a cancer; the cell may be a cancer cell.

The cancer can be, but are not limited to the following cancers: a leukemia cancer, a lymphoma, a lung cancer, a breast cancer, an ovarian cancer, a cervical cancer, a human glioma, a melanoma, a glioblastoma, a nasopharyngeal cancer, a liver cancer, a brain cancer, a pancreatic cancer, an uterine cancer, a testicular cancer, a skin cancer, a stomach cancer, a colon cancer, a bladder cancer or a rectal cancer.

The present invention also provides a method of inhibiting cell proliferation for non-therapeutic or therapeutic purposes, comprising: administrating the mRNA molecule to cells expressing the protein of interest to achieve inhibition of the cell proliferation. The cell can be a microbial cell, a plant cell, or an animal cell. The animal cell can be cancer cells, including but not limited to the following cancer cells: leukemia cancer, lymphoma, lung cancer, breast cancer, ovarian cancer, cervical cancer, human glioma, melanoma, glioblastoma, nasopharyngeal cancer, liver cancer, brain cancer, pancreatic cancer, uterine cancer, testicular cancer, skin cancer, stomach cancer, colon cancer, bladder cancer or rectal cancer.

The present invention also provides a method of promoting cell apoptosis for non-therapeutic or therapeutic purposes, comprising: administrating the mRNA molecule to cells expressing the protein of interest to promote cell apoptosis.

The cells can be microbial cells, plant cells, or animal cells. The animal cells can be cancer cells, including but not limited to the following cancer cells: leukemia cancer, lymphoma, lung cancer, breast cancer, ovarian cancer, cervical cancer, human glioma, melanoma, glioblastoma, nasopharyngeal cancer, liver cancer, brain cancer, pancreatic cancer, uterine cancer, testicular cancer, skin cancer, stomach cancer, colon cancer, bladder cancer or rectal cancer.

### Beneficial Effects

The m-PROTAC of the present invention has the following advantages: (1) the biological molecular properties of the medicament itself have higher safety, and in situ expression in the cytoplasm, can achieve the effect of high concentration expression therapy with low concentration administration; (2) the medicament can enhance the anti-tumor efficacy of peptide PROTACs, solve the problem of key cell penetration, and fully utilize its specificity advantages; (3) the medicament utilizes the PROTACs method to act in a catalytic like manner, achieving highly efficient targeted degradation function; (4) the medicament provides a new molecular form of targeted protein degradation and also offers new ideas for mRNA therapy applications.

A further detailed description of the present invention is provided in conjunction with specific implementation methods as below. The provided examples are only intended to illustrate the present invention and not to limit its scope. The examples provided below can serve as a guide for those skilled in the art to make further improvement and do not in any way limit the present invention.

### Brief Description of the Drawings

Figure 1 shows a schematic diagram of composition of m-PROTAC and degradation of a protein of interest.
Figure 2 is a schematic diagram of a v-m-PROTAC vector. Among them, Peptide represents the functional polypeptides targeted degrading of ER α.
Figure 3 shows the eGFP expression detection of m-PROTAC by flow cytometry analysis.
Figure 4 shows images of MCF-7 cells transfected with 1 µg/mL m-PROTAC for 24 hours. The upper left corner shows the white light channel map of cells treated with m-PROTAC, the lower left corner shows the white light channel map of untreated cells, the upper right corner shows the green fluorescence channel map of cells treated with m-PROTAC, and the lower right corner shows the green fluorescence channel map of untreated cells; Bar=20µm_{∘}
Figure 5 shows the expression levels of ER α in MCF-7 cells treated with different concentrations of mRNA for 24 hours.
Figure 6 shows the expression levels of ER α in MCF-7 cells treated with 1 µg/mL mRNA for different durations.
Figure 7 shows the expression levels of ER α under co-incubation of MG-132 and m-PROTAC. In the figure on the right, m represents m-PROTAC.
Figure 8 shows the cell viability of MCF-7 cells treated with m-PROTAC at different concentrations for 24 or 48 hours.
Figure 9 shows the apoptosis analysis results of MCF-7 cells treated with m-PROTAC at different concentrations.
Figure 10 shows the cell cycle analysis results of MCF-7 cells treated with m-PROTAC at different concentrations.
Figure 11 is a schematic diagram of v-m-PROTAC-2 vector. Among them, Peptide-2 represents the functional polypeptides targeted degrading of BCL-x_{L} .
Figure 12 shows targeted degradation of BCL-x_{L} by m-PROTAC-2 in MDA-MB-231 cells changes with concentration.
Figure 13 shows characterization results of m-PROTAC-2/LNPs. (A) Zeta potential distribution of m-PROTAC-2/LNPs, (B) particle size distribution (intensity) of m-PROTAC-2-LNPs, (C) analysis of Zeta potential measurement, (D) analysis of particle size measurement.
Figure 14 shows images of mice treated with luciferase mRNA LNPs or PBS. (A) In vivo bioluminescence images of mice treated with luciferase mRNA LNPs or PBS for 3 or 6 hours (n=3), (B) ex vivo bioluminescence images of tumors and major organs treated with luciferase mRNA LNPs or PBS for 6 hours.
Figure 15 shows anti-tumor activity of m-PROTAC-2 in a 4T1 xenograft animal model. (A) In vivo treatment protocol for 4T1 mouse model; when treated with m-PROTAC-2/LNP (0.5mg/kg), LNPs, or PBS, (B) changes in body weight over time in the 4T1 mouse model, (C) changes in tumor volume over time in the 4T1 mouse model, (D) photos of tumors isolated from the mice, and (E) weights of tumors isolated from the mouse model.

### Detailed Description

The experimental methods in the following examples, unless otherwise specified, are all conventional methods. The materials, reagents, instruments, etc. used in the following examples can be obtained from commercial sources unless otherwise specified. The quantitative experiments in the following examples were conducted with three replicates, and the results were averaged. In the following examples, unless otherwise specified, the first position of each nucleotide sequence in the sequence table is the 5 'terminal nucleotide of the corresponding DNA, and the last position is the 3' terminal nucleotide of the corresponding DNA.

### Example 1: Preparation of m-PROTAC

This example provides an mRNA PROTAC molecule for targeted degradation of ER alpha, abbreviated as m-PROTAC, which functions by encoding a functional polypeptide (referred to as E3-ER alpha). The functional peptide E3-ER alpha is obtained by sequentially connecting a heptapeptide sequence (PIYPALA, SEQ ID No.1) targeting VHL E3 ubiquitin ligase moiety, a linking peptide sequence (GSGS, SEQ ID No.2), and a peptide sequence (QLLRHLILH, SEQ ID No.4) targeting ER alpha protein binding, its sequence is SEQ ID No.6 in the sequence listing.

A 5' modified cap structure (Cap) of m-PROTAC is obtained by sequentially ligating a 5' non-coding region of β-globin, a Kozak element, a coding region (obtained by sequentially connecting enhanced green fluorescent protein (eGFP), P2A element, and mRNA of functional polypeptide E3-ER α), 3' non-coding region of α-globin, and polyadenylic acid structure (Poly-A). The sequence of m-PROTAC is SEQ ID No.7 in the sequence listing, and the DNA sequence of corresponding m-PROTAC is SEQ ID No.8 in the sequence listing.

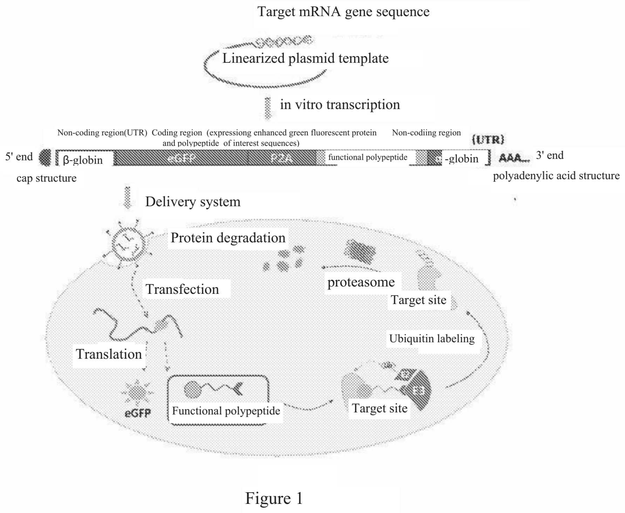

The principle of m-PROTAC composition and degradation of a protein of interest is shown in Figure 1: mRNA of m-PROTAC is obtained by in vitro transcription from a linearized plasmid template of DNA containing m-PROTAC.

### 1. Preparation of recombinant vectors

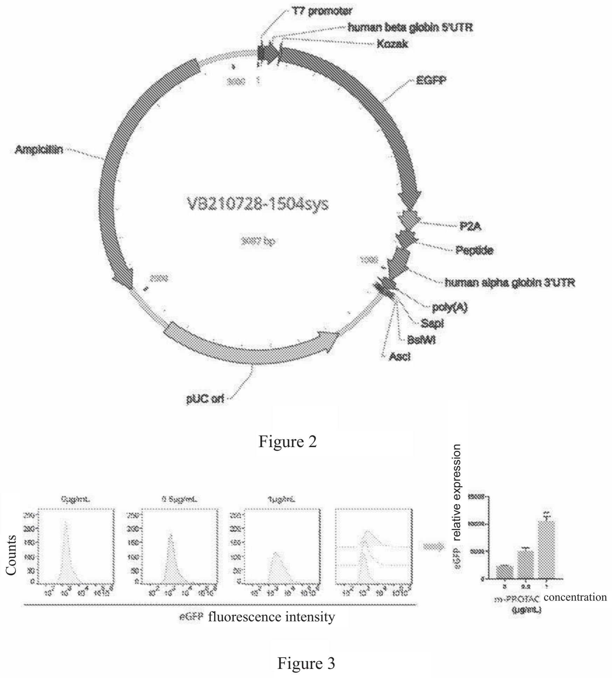

Based on the m-PROTAC template sequence shown in SEQ ID No. 8, an in vitro transcription vector v-m-PROTAC (its sequence is SEQ ID No. 9 in the sequence listing, as shown in Figure 2) containing the m-PROTAC template sequence shown in SEQ ID No. 8 was synthesized. The in vitro transcription promoter is a T7 promoter, and the downstream contains an AscI enzyme cleavage site.

The linear plasmid v-m-PROTAC was obtained by cleavage with AscI enzyme and purification.

### 2. In vitro transcription of m-PROTAC

1) Based on the manufacturer's protocol of the in vitro transcription reagent (Promega, P1300), add the followings in order into a 1.5mL RNase free centrifuge tube:
   ① 5×T7 transcription buffer: 20.0 µL
   ② rNTP mix: 30.0 µL
   ③ 1-2 µg linear plasmid (dissolved in DEPC water): 32.5 µL
   ④ Cap modified guanine (40mM): 7.5 µL
   ⑤ Enzyme mix: 10.0 µL;
2) Gently mix with a pipette and react in a 37°C metal bath for 4-6 hours;
3) Purification: RQ1 RNase Free DNase was added at a concentration of 1U/µg DNA template, incubated at 37°C for 30 minutes, then purified using mRNA purification kit to obtain m-PROTAC. The concentration was measured and packaged as aliquots for storage in a -85°C refrigerator.

### Example 2: Cells transfected with m-PROTAC can efficiently express the protein of interest

MCF-7 cells of breast cancer cell line were cultured in a 12 well plate using DMEM medium and transfected with m-PROTAC of different concentrations (the concentration of m-PROTAC in the system was set to 0, 0.5, 1 µg/mL). After incubating at 37°C for 4 hours, cells were washed with PBS to replace DMEM complete medium and incubation was continued for 20 hours (the total incubation time was 24 hours). Then cells were collected for flow cytometry to detect the expression level of eGFP in cells. The quantitative analysis results were shown in Figure 3, where the intensity of expressing green fluorescent protein at 1 µ g/mL is 4.44 times that of the control group.

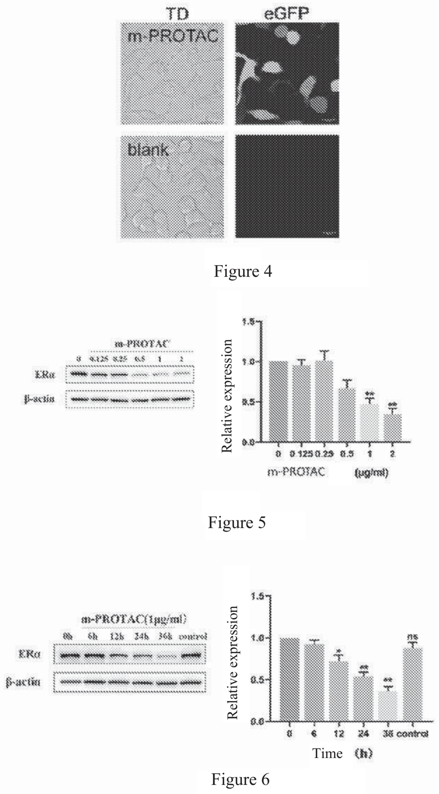

Cell imaging was performed on MCF-7 cells transfected with 1 µg/mL m-PROTAC, and the results were shown in Figure 4. It showed that the transfected cells produced significant green fluorescence of eGFP., indicating that cells transfected with m-PROTAC could efficiently express the protein of interest.

### Example 3: Targeted degradation of ER α by m-PROTAC in MCF-7 cells changes with concentration

MCF-7 cells of breast cancer cell line were cultured in a 12 well plate using DMEM medium. MCF-7 cells were transfected with m-PROTAC of different concentrations (the concentration of m-PROTAC in the system was set to 0, 0.125, 0.25, 0.5, 1, and 2 µg/mL). After incubating at 37°C for 4 hours, cells were washed with PBS to replace DMEM complete medium and incubation was continued for 20 hours (the total incubation time was 24 hours). Then cells were collected for Western Blot analysis. The control protein was β-actin gene. The primary antibody used was ER α rabbit monoclonal antibody (Cell Signaling Technology, # 8644) and β- actin rabbit monoclonal antibody (ABclonal, AC026).

The result was shown in Figure 5. The expression level of ERα showed a significant decrease with the increase of mRNA incubation concentration, indicating that ERα has been degraded, and the degradation is mRNA concentration dependent. The degradation efficiency reached 60% at 1 µ g/mL, and then 1 µ g/mL mRNA was selected for exploration.

### Example 4: Targeted degradation of ER α by m-PROTAC in MCF-7 cells over time

MCF-7 cells of breast cancer cell line were cultured in a 12 well plate using DMEM medium. MCF-7 cells were transfected with m-PROTAC (the concentration of m-PROTAC in the system was 1 µg/mL). After the transfection time reached 0, 6, 12, 24 or 36 hours at 37 °C, the cells were collected for Western Blot analysis. Cells treated with control mRNA at a concentration of 1 µg/mL for 24 hours as a control. The control mRNA is an mRNA that does not contain a functional polypeptide encoding sequence, prepared according to the following method: according to the method of step 1 in Example 1, the DNA fragment shown in SEQ ID No.8 in the v-m-PROTAC obtained in Example 1 was replaced with SEQ ID No.10, and a recombinant vector v-control was obtained; in accordance with the method of step 1 in Example 1, v-m-PROTAC was replaced with v-control, all other steps remained unchanged, and the control mRNA was obtained.

The result was shown in Figure 6. The expression level of ERα showed a significant reduction with the increase of incubation time, indicating that the degradation of ERα is mRNA incubation time dependent. The degradation efficiency at 24 hour reached 60%, and a 24-hour incubation time was selected for further experiments.

### Example 5: m-PROTAC degrades ERα by the ubiquitin-protease system in MCF-7 cells

MG-132 can inhibit proteasome activity and is a common proteasome inhibitor.

Breast cancer cell line MCF-7 cells were cultured in DMEM medium in a 12 well plate, and MG-132 was added to the culture system. The concentration of MG-132 in the culture system was 10 µM; MCF-7 cells were transfected with m-PROTAC (the concentration of m-PROTAC was set as 1 µ g/mL in the system). After incubating at 37°C for 4 hours, cells were washed with PBS to replace DMEM complete medium containing 10 µ M MG132 and incubation was continued for 20 hours (the total incubation time was 24 hours). Then cells were collected for Western Blot analysis. Untreated cells (blank), cells treated with m-PROTAC only, and cells treated with only MG-132 were set as controls, respectively.

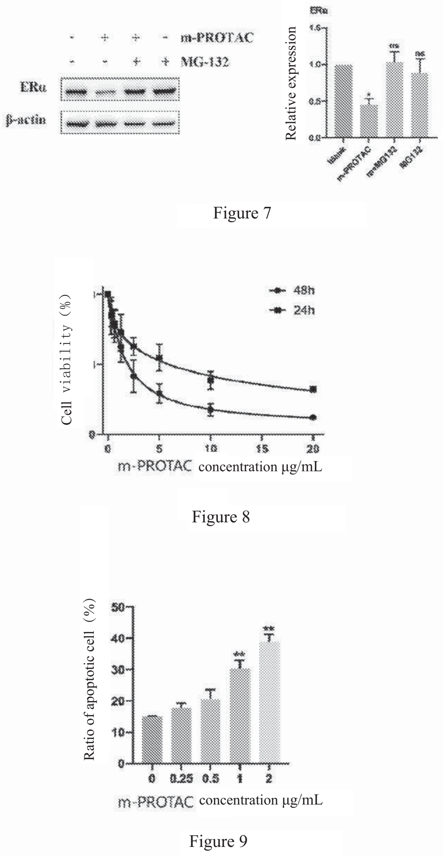

The result was shown in Figure 7. After adding MG-132, there was no significant reduction in the expression level of ERα, while the expression level of ER α in cells without MG-132 treatment reduced significantly, indicating that MG-132 can block degradation of ERα by m-PROTAC, thereby validating that m-PROTAC induced ERα degradation is carried out by the ubiquitin protease system.

### Example 6: m-PROTAC inhibits proliferation of MCF-7 cells

MCF-7 cells of breast cancer cell line were cultured in a 12 well plate using DMEM medium. MCF-7 cells were transfected with m-PROTAC of different concentrations (the concentration of m-PROTAC in the system was set to 0, 0.3125, 0.625, 1.25, 2.5, 5, 10, 20 µg/mL). After incubating at 37°C for 4 hours, medium was replaced with DMEM complete medium and incubation was continued for 20 hours (the total incubation time was 24 hours) or 44 hours (the total incubation time was 48 hours), and then the cell activity was detected. Changes of cell viability were detected using a CCK-8 assay kit (Biyuntian, C0038).

The results were shown in Figure 8, m-PROTAC has a significant inhibitory effect on proliferation of MCF-7 cells. IC50 at 24 hours is about 8.2 µg/mL, and the IC50 of treatment for 48 hours is about 1.7 µg/mL, corresponding to molar concentrations of 24.5 nM and 5.1 nM. The currently reported ER α PROTACs are all at the micromolar level. This indicates that a certain concentration of m-PROTAC can effectively kill MCF-7 cells. The m-PROTAC of the present invention has a higher efficiency in targeted degradation of proteins of interest compared to other PROTACs systems.

### Example 7: m-PROTAC results in apoptosis of MCF-7 cells through S phase arrest

MCF-7 cells of breast cancer cell line were cultured in a 12 well plate using DMEM medium. MCF-7 cells were transfected with m-PROTAC of specific concentrations (the concentrations were set to 0, 0.125, 0.25, 0.5, 1, 2 µg/mL). After incubating at 37°C for 4 hours, cells were washed with PBS to replace DMEM complete medium and incubation was continued for 20 hours (the total incubation time was 24 hours). Then cells were collected for apoptosis analysis.

For apoptosis analysis, following manufacturer's protocol of Annexin V-FITC apoptosis detection kit (Beyotime, C1062), the cells were stained with Annexin V-FITC and PI. For cell cycle analysis, cells were resuspended in a 70% precooled ethanol aqueous solution, left overnight at 4 ° C, and then centrifuged to remove ethanol. According to the instructions of the Cell Cycle and Apoptosis Analysis Kit (Beyotime, C1052), the collected cells were treated with RNase A and propidium iodide (PI). Finally, the stained samples were tested using flow cytometer and analyzed using ModFit LT 5.0. For each individual experiment, over 10000 cells were counted for cell apoptosis and cycle detection.

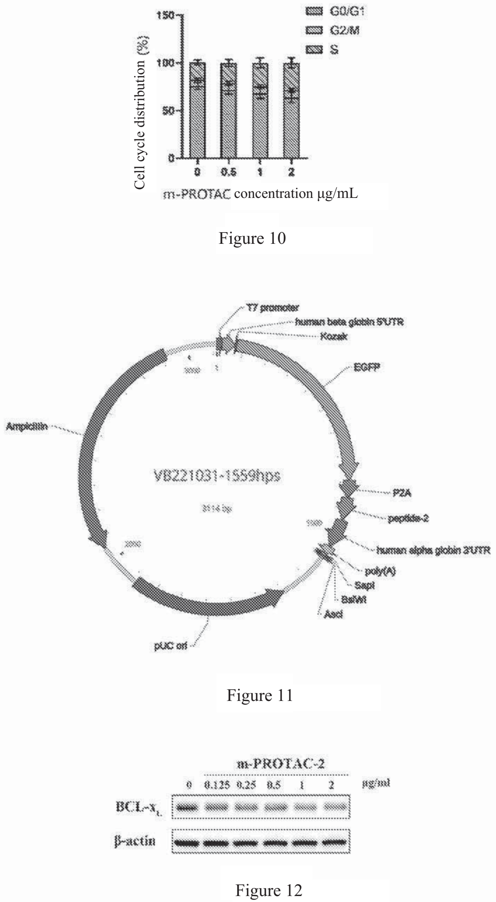

The results were shown in Figures 9 and 10. As the concentration increases, the proportion of late stage apoptosis in MCF-7 cells treated with m-PROTAC increases, and the cycle arrest in the S phase increases, indicating that m-PROTAC leads to MCF-7 cell apoptosis through S phase arrest.

### Example 8: Preparation of m-PROTAC-2 and Its Targeted Degradation of BCL-xL in MDA-MB-231 Cells with Concentration Variation

This example provides an mRNA PROTAC molecule for targeted degradation of BCL-x_{L}, abbreviated as m-PROTAC-2, which functions by encoding a functional polypeptide (referred to as E3-BCL-x_{L}). The functional polypeptide E3-BCL-x_{L} is obtained by sequentially connecting a heptapeptide sequence (PIYPALA, SEQ ID No.1) targeting the VHL E3 ubiquitin ligase meioty, a linking peptide sequence (GGGGGG, SEQ ID No.3), and a polypeptide sequence (GQVGRQLAIIGDAINR, SEQ ID No.5) targeting BCL-x_{L} protein binding, its sequence is SEQ ID No.11 in the sequence listing.

A 5' modified cap structure (Cap) of m-PROTAC-2 is obtained by ligating a 5' non-coding region of β- globin, a Kozak element, a coding region (obtained by sequentially connecting an enhanced green fluorescent protein (eGFP), a P2A element, and an mRNA of functional polypeptide E3-BCL-x_{L}), 3' non-coding region of α - globin, and polyadenylic acid structure (Poly-A). The sequence of m-PROTAC-2 is SEQ ID No. 12 in the sequence listing, and a DNA sequence of the corresponding m-PROTAC-2 is SEQ ID No. 13 in the sequence listing.

m-PROTAC-2 was obtained by in vitro transcription from a linearized plasmid template containing DNA containing m-PROTAC-2.

Based on the m-PROTAC-2 template sequence shown in SEQ ID No. 13, an in vitro transcription vector v-m-PROTAC-2 (its sequence is SEQ ID No. 14 in the sequence listing, as shown in Figure 11) containing the m-PROTAC-2 template sequence shown in SEQ ID No. 13 was synthesized. The in vitro transcription promoter is a T7 promoter, and the downstream contains an AscI enzyme cleavage site.

The linear plasmid v-m-PROTAC-2 was obtained by digestion with AscI enzyme and subsequent purification.

In accordance with the method of step 2 in Example 1, v-m-PROTAC-2 was replaced with v-m-PROTAC-2 to obtain m-PROTAC-2.

MDA-MB-231 cells of breast cancer cell line were cultured in L-15 medium in a 12-well plate. MDA-MB-231 cells were transfected with m-PROTAC-2 of different concentrations (the concentration of m-PROTAC-2 in the system was set to 0, 0.125, 0.25, 0.5, 1, 2 µg/mL). After incubation at 37 °C for 4 hours, cells were washed with PBS to replace L-15 complete medium for further 20 hours incubation (the total incubation time was 24 hours). Cells were collected for Western Blot analysis. The control protein was β -actin gene. The primary antibodies used were BCL-x_{L} recombinant rabbit monoclonal antibody (Yeasen Biotechnology, 31011ES) and β-actin rabbit monoclonal antibody (ABclonal, AC026).

The results were shown in Figure 12. BCL-x_{L} showed a significant decrease in expression levels with increasing mRNA incubation concentration, indicating that BCL-x_{L} was degraded and the degradation was mRNA concentration dependent.

### Example 9: m-PROTAC-2 inhibits tumor growth in vivo

In order to confirm the anti-tumor ability of m-PROTAC, 4T1 cell line (mouse breast cancer cell line) overexpressing BCL-x_{L} was used for xenograft experiment.

BALB/c mice used in this example were products from Guangdong Medical Experimental Animal Center. 4T1 mouse tumor model was obtained by subcutaneous injection of 1 × 10⁵ 4T1 cells into each of 4-week-old female BALB/c mice to get a tumor size of 100mm³. All animal experiments were conducted in accordance with the guidelines and approvals of the Institutional Animal Care and Use Committee of Tsinghua University Shenzhen International Graduate School and Guangdong Provincial Center for Medical Laboratory Animal Management in China (License Number: 2023).

The present invention selected cationic lipid nanoparticles (LNPs) SM-102 (Neocura Bio-Medical Technology Co., Ltd., CAS: 2089251-47-6) as the in vivo delivery carrier for m-PROTAC. Cationic lipid nanoparticles SM-102, distearoyl phosphatidylcholine (DSPC), cholesterol, and DMG-PEG2000 were dissolved in ethanol at a molar ratio of 50:10:38.5:1.5 and mixed by shaking well to prepare lipid mixture. Firefly luciferase mRNA (i.e. Luciferase mRNA, whose sequence is shown as SEQ ID No. 15 in the sequence listing) or m-PROTAC-2 was diluted in 50mM citrate buffer (pH 4) to obtain mRNA aqueous solution. By using a microfluidic device (nanoE, Micro&Nano), the lipid mixtures were mixed with mRNA aqueous solutions in a volume ratio of 1:3, dialyzed in PBS (pH 7.4) for 18 hours to remove ethanol and complete the citrate buffer exchange process, to form mRNA/LNPs (i.e. m-PROTAC-2/LNPs or Luciferase mRNA/LNPs).

According to the above method, LNPs carriers were obtained by same steps except for not adding mRNA.

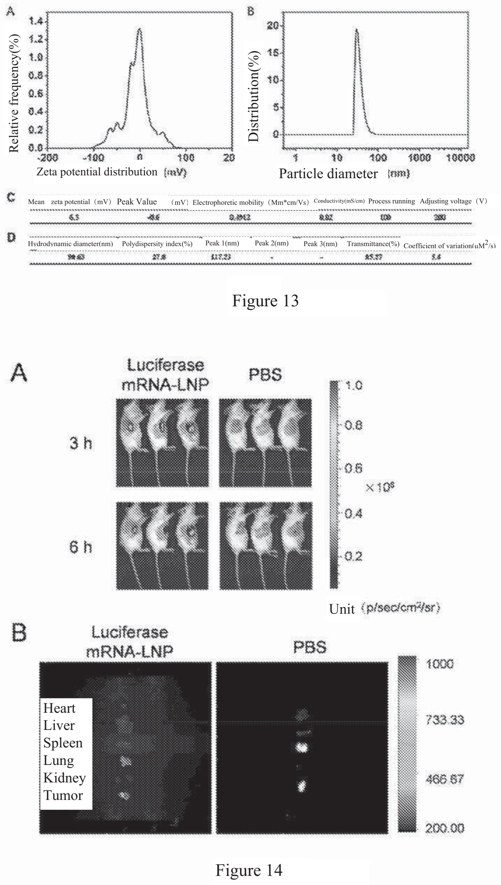

Size distribution, polydispersity index (PDI), and zeta potential (Zeta) of mRNA/LNPs were measured through Litesizer ^{™} 500 instrument (Anton Paar). The encapsulation efficiency of lipid nanoparticles was evaluated using RiboGreen method (characterization results were shown in Figure 13).

The results showed that the particle size distribution exhibited a unimodal pattern with a uniform distribution.

### (1) Validation experiment of luciferase mRNA

After successfully establishing a tumor bearing mouse model, Luciferase mRNA (i.e. Luciferase mRNA/LNPs) encapsulated in SM-102 was intratumorally injected into the 4T1 mouse tumor model. Three or six hours after injection, the bioluminescence signal was recorded using an in vivo imaging instrument (IVIS Spectrum, PerkinElmer). After 6 hours, one mouse from each group of mice was subjected to dissection. Tumors and major organs (heart, liver, spleen, lungs, and kidneys) were collected for ex vivo imaging (In Vivo F Pro, Bruker). Mice administered only PBS served as the control group.

Results were shown in Figure 14, the luciferase signal of the mRNA LNP group of mice was clear, confirming effective transfection of mRNA by SM-102. After 6 hours, the signal distributions in the tumor and major organs (heart, liver, spleen, lungs, and kidneys) were analyzed, showing that mRNA was only effectively delivered and expressed within the tumor.

### (2) m-PROTAC inhibits tumor growth in vivo

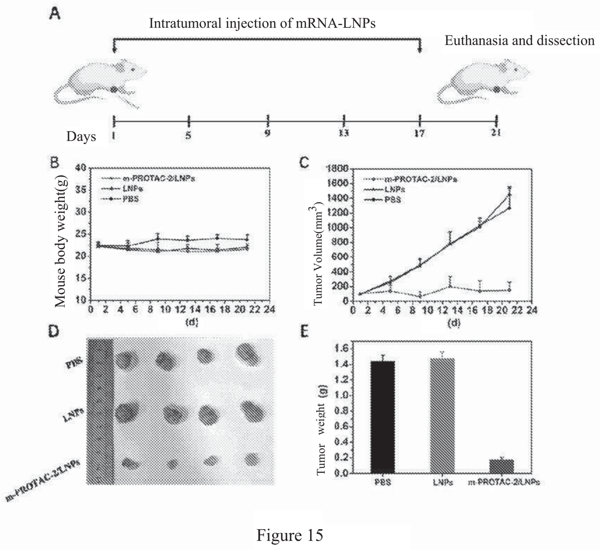

To verify the efficacy of m-PROTAC in vivo, 4T1 mouse tumor models with a tumor volume of approximately 100 mm³ was randomly divided into three groups, with four mice in each group. Equal volumes of PBS, LNPs carrier, or m-PROTAC-2/LNP (0.5mg/kg) were intratumorally injected into the 4T1 mouse tumor model every four days, respectively. The in vivo treatment protocol for the 4T1 mouse tumor model was shown in Figure 15 (A). Tumor volume and mouse weight were measured before each injection, and the tumor volume was calculated using the formula V=1/2ab2 (where a is the maximum diameter and b is the minimum diameter). Monitoring was continued for 4 days after the last injection, and then the mouse tumor model was euthanized with CO₂. Tumor and major organs of the 4T1 mouse tumor model were collected, weighed and the tumor volume was measured. The results were shown in Figure 15, the 4T1 mouse tumor model treated with m-PROTAC-2/LNPs exhibited consistent and stable body weight throughout the experiment as the control group treated with LNPs carrier and PBS. At the same time, the m-PROTAC-2/LNPs group showed significant tumor growth inhibition (B and C in Figure 15). This indicates that m-PROTAC-2 has low toxicity and high anti-tumor capacity. After dissection, the tumors in the 4T1 mouse tumor model group treated with m-PROTAC-2/LNPs were significantly smaller than those in the control group (D and E in Figure 15). In addition, the effective dose of m-PROTAC-2 in vivo is significantly lower than that of 4T1 mouse tumor models treated with DT2216 (a novel BCL-xL specific PROTAC, 15 mg/kg), demonstrating superior tumor treatment efficacy. These results demonstrate the effectiveness of m-PROTAC in vivo and its enormous potential in the treatment of related diseases.

### Industrial Practicality

The m-PROTAC of the present invention can efficiently target and degrade proteins of interest, and can be used for treating and/or preventing diseases/lesions mediated by proteins of interest. It can also be used for inhibiting tumor cell proliferation or promoting tumor cell apoptosis, achieving tumor treatment.

## Claims

1. An mRNA molecule for targeted protein degradation, **characterized in that** the mRNA molecule sequentially comprises a 5' cap structure, a 5' non-coding region, a Kozak element, a coding region, a 3' non-coding region, and a a polyadenylic acid structure, the coding region comprises a functional polypeptide coding gene, and the functional polypeptide comprises a VHL E3 ubiquitin ligase targeting peptide, a linking peptide, and a protein of interest targeting polypeptide which are sequentially connected.

2. The mRNA molecule for targeted protein degradation according to claim 1, **characterized in that** the VHL E3 ubiquitin ligase targeting peptide has a sequence shown in SEQ ID No.1;
the linking peptide has a sequence shown in SEQ ID No.2 or SEQ ID No.3;
the protein of interest targeting polypeptide is a polypeptide that specifically targets the protein of interest, and has a sequence shown in SEQ ID No.4 or SEQ ID No.5.

3. The mRNA molecule for targeted protein degradation according to claim 2, **characterized in that** the functional polypeptide has a sequence shown in SEQ ID No. 6 or SEQ ID No. 11.

4. The mRNA molecule for targeted protein degradation according to any one of claims 1-3, **characterized in that** the 5' non-coding region is 5' non-coding region of β-globin, and the 3' non-coding region is 3' non-coding region of α-globin.

5. The mRNA molecule for targeted protein degradation according to any one of claims 1-3, **characterized in that** the coding region further comprises another functional protein coding gene or a reporter protein coding gene, and a spacer region set between them and the functional polypeptide.

6. The mRNA molecule for targeted protein degradation according to any one of claims 1-3, **characterized in that** the mRNA molecule has a sequence shown in SEQ ID No. 7 or SEQ ID No. 12.

7. A DNA molecule encoding the mRNA molecule of any one of claims 1-5, wherein the DNA molecule has a sequence shown in SEQ ID No. 8 or SEQ ID No. 13.

8. A recombinant vector comprising the DNA molecule according to claim 7.

9. A recombinant microorganism comprising the mRNA molecule of any one of claims 1-6, the DNA molecule of claim 7, or the recombinant vector of claim 8.

10. A cell comprising the mRNA molecule of any one of claims 1-6, the DNA molecule of claim 7, the recombinant vector of claim 8, or the recombinant microorganism of claim 9.

11. Use of any of the mRNA molecule according to any one of claims 1-6, the DNA molecule according to claim 7, the recombinant vector according to claim 8, or the recombinant microorganism according to claim 9:
M1) preparation a product for treating and/or preventing diseases/lesions mediated by the protein of interest according to any one of claims 1-6;
M2) treatment and/or prevention of diseases/lesions mediated by the protein of interest;
M3) preparation of a product that inhibits cell proliferation;
M4) inhibition of cell proliferation;
M5) preparation of a product that promotes cell apoptosis;
M6) promotion of cell apoptosis.

12. The use according to claim 11, **characterized in that**, the disease mediated by the protein of interest is a cancer; the cell is a cancer cell.

13. The use according to claim 12, **characterized in that**, the cancer is a leukemia cancer, a lymphoma, a lung cancer, a breast cancer, an ovarian cancer, a cervical cancer, a human glioma, a melanoma, a glioblastoma, a nasopharyngeal cancer, a liver cancer, a brain cancer, a pancreatic cancer, an uterine cancer, a testicular cancer, a skin cancer, a stomach cancer, a colon cancer, a bladder cancer or a rectal cancer.

14. A method for degrading a protein of interest, comprising: applying the mRNA molecule according to any one of claims 1-6 to a biological cell, a tissue, or an organ expressing the protein of interest, to achieve degradation of the protein of interest in the biological cell, tissue, or organ.

15. The method for degrading a protein of interest according to claim 14, **characterized in that** the biological cell is a microbial cell, a plant cell, or an animal cell.

16. A method for treating and/or preventing a disease/lesion mediated by a protein of interest, comprising: administering the mRNA molecule according to any one of claims 1-6 to an animal body suffering from a disease/lesion mediated by the protein of interest, to achieve treatment and/or prevention and/ or alleviation of the disease/lesion mediated by the protein of interest in the animal body.

17. The method according to claim 16, **characterized in that**, the disease mediated by the protein of interest is a cancer; the cell is a cancer cell.

18. The method according to claim 17, **characterized in that**, the cancer is a leukemia cancer, a lymphoma, a lung cancer, a breast cancer, an ovarian cancer, a cervical cancer, a human glioma, a melanoma, a glioblastoma, a nasopharyngeal cancer, a liver cancer, a brain cancer, a pancreatic cancer, an uterine cancer, a testicular cancer, a skin cancer, a stomach cancer, a colon cancer, a bladder cancer or a rectal cancer.

19. A method for inhibiting cell proliferation, comprising: administrating the mRNA molecule according to any one of claims 1-6 to cells expressing a protein of interest to achieve inhibition of the cell proliferation.

20. A method for promoting cell apoptosis, comprising: administrating the mRNA molecules according to any one of claims 1-6 to cells expressing a protein of interest, to achieve promotion of the cell apoptosis.
